# EUROPEAN PATENT APPLICATION

(11) **EP 1 374 867 A1**
(43) Date of publication of application: **02.01.2004**
(21) Application number: 03013823.4
(22) Date of filing: 18.06.2003
(51) Int. Cl.: A61K 31/4706, A61P 31/18

(54) **Use of chloroquine, hydroxychloroquine and 4 amino-quinolinic derivatives to obtain a drug for the anti retroviral therapy, active towards hiv sensitive strains and towards hiv strains which are resistant to both nucleosidic and non-nucleosidic reverse transcriptase inhibitors and to proteases inhibitors**

(30) Priority: 28.06.2002 IT BO20020416
(71) Applicant: Valpharma S.A., 47899 Serravalle (SM)
(72) Inventor: Valducci, Roberto, 47039 Savignano Sul Rubicone (FC) (IT); Alighieri, Tiziano, 47900 Rimini (IT); Avanessian, Serozh, 47900 Rimini (IT)
(74) Representative: Negrini, Elena

(57) **Abstract**

Use of at least one 4 amino-quinolinic derivative to obtain a drug for the anti-retroviral therapy, especially in the therapy of the HIV disease-infection caused by retroviral strains, which are sensitive and/or resistant to known therapies.

Said 4 amino-quinolinic derivative is chloroquine and/or hydroxychloroquine and the drug can be orally administered once or several times a day.

## Description

The present invention concerns the pharmacological and therapeutic field and, in particular, relates to the use of chloroquine, hydroxychloroquine and 4 amino-quinolinic derivatives to obtain an anti-retroviral drug, active towards HIV sensitive strains and towards HIV strains which are resistant to the current anti-HIV therapies.

The activity of chloroquine *versus* HIV was first described by Tsai (1990), who demonstrated that this substance inhibits the HIV replicative cycle in continuous cultures of cells from the lymphocyte system by interfering with post-transcriptional processes. These *in vitro* results were confirmed in some following studies, in which the inhibition of the HIV replicative cycle was observed either using hydroxychloroquine or treating the cell cultures with chloroquine in a concentration similar to that achievable in plasma of patients treated with this drug for a long time (Sperber, 1993; Chiang, 1996).

The research of Sperber (1993), carried out using PBMC and continuous cell lines derived from lymphocyte T (CEM) and monocyte (U-937), clearly underlined that hydroxychloroquine inhibits the HIV-1 replication (> 75%), both in the primary cell lines (PBMC) and in the continuous lines of cells with a mono-lymphocyte origin (U-937; CEM). Using hydroxychloroquine at concentration suitable for inhibit viral replication, no toxic effect was found in cell cultures. Moreover, hydroxychloroquine almost completely inhibits the production of infecting viral particles: in fact viral preparations, obtained from cell cultures treated with hydroxychloroquine, rarely infect CEM cells used as target.

Working *in vitro,* Boelaert *et al*. (1999) showed that the addition of hydroxychloroquine to the ddI (didanosine) *plus* HU (hydroxyurea) association results in an extra effect for the inhibition of the HIV replication.

Substantially analogous results were reported by Boelaert *et al.* (2001) with regard to the additional effect obtained *in vitro* when hydroxychloroquine was added to the association AZT *plus* HU.

Lange (1994) showed that plasmatic concentrations of hydroxychloroquine ranging from 7 to 15 microM, ever if prolonged over 18-24 months, rarely induced retinopathy.

The first clinical trial, suitable for evaluating the anti-retroviral effectiveness of hydroxychloroquine, was carried out by Sperber *et al.* (1995).

Forty asymptomatic HIV-1 positive patients, characterised by a number of T CD4+/mm³ cells ranging from 200 and 500, were observed; the analysis was conducted using double-blind randomisation. Patients were randomly organised in order to receive either hydroxychloroquine (800 mg/day) or a placebo for 8 weeks. At the end of this study the quantity of HIV-RNA significantly decreased in the arm treated with hydroxychloroquine, while increased if compared to the basal value in the arm treated with the placebo. No negative event was observed in the arm treated with hydroxychloroquine.

In an other preliminary clinical trial (Sperber *et al.* 1997), carried out on 38 HIV+ patients characterised by a number of CD4/mm³ cells ranging from 200 to 500, the administration of 800 mg/day hydroxychloroquine determined a significant reduction of the plasmatic HIV-RNA in the group of patients who assumed hydroxychloroquine if compared to the group that represented the control (placebo).

These preliminary observations have brought to a second 16 weeks randomised trial, aimed to compare the effectiveness of hydroxychloroquine *versus* AZT in 72 asymptomatic patients characterised by a number of CD4 cells/mm³ ranging from 200 to 500.

Patients have been randomised in order to receive 800 mg/day hydroxychloroquine (n = 35) and 500 mg/day AZT (n = 37). Every patient completed the 16 weeks study and no negative events were observed both in the arm with hydroxychloroquine and in that with AZT.

Patients from both the groups showed a significant reduction of HTV-RNA, seric p24 and plasmatic infecting virus titre after 16 weeks of study. Although in this study the global data analysis pointed out a higher effectiveness of AZT in the reduction of the plasmatic viremia, 8 patients showed, at the end of the treatment with AZT, an increase of the plasmatic viremia. Said eventuality suggested the appearance, during therapy with AZT, of viral variants, which are resistant to the drug.

On the contrary, the anti-retroviral activity of hydroxychloroquine was found to be longer and nobody of the patients essayed by this drug showed an increase of plasmatic viremia at the 8th and at the 16th week. These experimental data could point out that the appearance of the resistance towards hydroxychloroquine is a phenomenon relatively difficult to be set up.

Recently (Paton *et al.* 2002) the antiviral activity of the association of hydroxychloroquine (400 mg/day) *plus* HU *plus* ddI was evaluated *in vivo:* the study was carried out for 48 weeks, in 22 patients with a HIV-1 asymptomatic infection, a plasmatic viremia higher than 10.000 copies *per* ml and a number of T CD4+ cells superior than 150 *per* mmc. The treatment was well tolerated and produced a 1,3 logs decrement of plasmatic HIV-RNA during the time-course of the study.

However, said studies and observations have neither defined nor furnished a therapeutic indication about the use, in the HIV infection-disease, of chloroquine and/or hydroxychloroquine, and in general of 4 amino-quinolinic derivatives. Said studies have completely ignored the viral strains which are partially or totally resistant to the current anti-AIDS therapies.

The drugs used in the therapy of the HIV infection-disease and known until now, belong mainly to two classes (nucleosidic and non-nucleosidic reverse transcriptase inhibitors and proteases inhibitors), whose mechanism of action is referable to the inhibition of two viral enzymes, the reverse transcriptase and the protease and, therefore, which easily select, in the viral genome, mutations that confer either intra-class or inter-class cross-resistance. These drugs have further disadvantages, such as to be expensive, to be characterised by a complicate administration and to have a profile of chronic toxicities which is poorly known.

The main object of the present invention is to propose the use of chloroquine, hydroxychloroquine and in general 4 amino-quinolinic derivatives to obtain a drug for the anti-retroviral therapy, capable to show a long-term inhibiting activity towards HIV viral replication in every cell which is a target for the infection and capable to inhibit, contextually and with equal effectiveness, the replication of the HIV strains, which are either sensitive or resistant to the current anti-retroviral therapies.

Other object is to propose the use of 4 amino-quinolinic derivatives, and in particular of chloroquine and hydroxychloroquine, to obtain a chemotherapic low-cost and orally assumable, which does not select, unless in a negligible rate, resistant viral variants and which does not show any activity against other drugs, used for the treatment of this infection.

Related to the last point, further object is to propose the use of the chloroquine, hydroxychloroquine and other 4 amino-quinolinic derivatives to obtain a drug capable to inhibit the maturation of the virions (structural alterations in the glycosilated portion of the gp120 of the viral pericapsid) and, therefore, which is characterised by a mechanism new and fundamentally different with respect to that of drugs known until now and, for this, that can be associate to said drugs, with an additional or synergetic activity.

The above-mentioned objects are achieved according to the claim content.

Hydroxychloroquine, chloroquine and 4-amino-quinolinic anti-retroviral activity is not limited to laboratory viral strains, but can also be observed towards clinical isolates such as X4, R5, X4 / R5 in monocyte and lymphocyte cultures, towards the subtype C of HIV-1, towards HIV-2 and all the known subtypes of HIV-1 (A-O). The inhibiting effect towards the subtype C of HIV-1 and towards HIV-2 is of particular interest considering the wide diffusion of these viruses in Africa.

Hydroxychloroquine, chloroquine and in general 4-amino-quinolinic derivatives are weak bases that can induce into the cell some alterations at the acidic vesicles level, which, by consequence, produce some dysfunction in essential enzymes, caused by post-translational modifications of the protein.

Indeed, weak bases, by increasing pH of the acidic vesicles, degrade different enzymes such as acidic hydrolases, and, therefore, inhibit structural modifications of the new formed viral proteins.

In the acidic vesicles the increase of the pH depends on the amount of chloroquine, hydroxychloroquine and 4-amino-quinolinic derivatives and the viruses obtained after multiple passages in cultures treated with chloroquine, hydroxychloroquine and 4-amino-quinolinic derivatives are not infective (Chiang *et al.* 1996). As chloroquine, hydroxychloroquine and 4-amino-quinolinic derivatives show an action (alteration of the glycosilated portion of gp120), which is completely different from that of known anti-retroviral chemotherapics (nucleosidic and non-nucleosidic reverse transcriptase inhibitors and proteases inhibitors), they can be used in association with all the known anti-retroviral chemotherapics.

Indeed, *in vitro* experiments of Boelaert *et al.* (1999, 2001) underlined an additional effect of chloroquine and hydroxychloroquine in association with AZT, ddI, HU on the HIV-1 replication.

As chloroquine, hydroxychloroquine and 4-amino-quinolinic derivatives have been used for long time, their toxicity profile is well known. The *in vivo* toxicity of CQ, HCQ and 4-amino-quinolinic derivatives can be observed at the ocular level (retinal maculopaty) and can be correlated with the cumulative dose more closely than with the daily dosage. The retinal maculopaty induced by chloroquine shows two phases: one, precocious and reversible, which is characterised, in most cases, by dischromie and alterations of the visual field and one, late and irreversible, in which there is the loss of the visus.

It is, however, possible to avoid the onset of this last effect by periodically submitting people treated by this substances to a test for the colours, which is easy to execute also by non specialists. If dischromie appears, it is necessary to suspend immediately the use of the drug, in order to prevent the evolution of the maculopaty to said irreversible phase. However it should be considered that there is a wide scientific documentation which underlines that, considering the dosage proposed in this report and all described clinical precautions, the incidence of the irreversible retinal maculopaty is negligible. Moreover, CQ and HCQ are usually administered by routine to pregnant women during the prophylaxis of malaria. With this dosage CQ and HCQ are well tolerated and they can be considered secure for both the mother and the foetus.

The dramatic diffusion of the AIDS in the Third World caught the attention of the scientific community towards therapies characterised by low cost, because the large majority of the countries affected by the epidemic can not sustain the high costs of the anti-retroviral therapy, which is now the most effective and which is based either on the association of 2 nucleosidic RT inhibitors and a proteases inhibitor or on 2 nucleosidic RT inhibitors and one non nucleosidic RT inhibitor (HAART).

For this and for other applications, chloroquine, hydroxychloroquine and 4-amino-quinolinic derivatives seem to have good characteristics, because they possess an anti-viral mechanism which is completely different with respect to that of chemotherapics used until now in the anti-HIV therapy.

Chloroquine and hydroxychloroquine act as inhibitors of the glycosilation of gp120 and some glycosilated portions of gp120 play a fundamental role in the HIV infectivity; this would explain the high decrease of infectivity of HIV produced by the use of chloroquine and hydroxychloroquine.

As chloroquine, hydroxychloroquine and the 4-amino-quinolinic derivatives show a new anti-HIV action, they can be suggested in order to control effectively the replication of HIV strains which are resistant to nucleosidic and non-nucleosidic reverse transcriptase and viral protease inhibitors in those patients characterised by viro-immunological failure, who represent 60-70% of the ones treated with HAART.

The strains of HIV-1, both sensitive to the therapy (isolated from patients that never were treated with anti-retroviral therapy) and resistant, which were used in the present research that originated the present innovation, were characterised genotypically and phenotypically to appraise the resistance to the nucleosidic and non-nucleosidic reverse transcriptase and protease inhibitors listed as follows.

**Nucleosidic inhibitors:** a) Zidovudine (AZT); b) Didanosine (ddI); c) Lamivudine (3TC); d) Stavudine (d4T); and) Abacavir (ABC).

**Non nucleosidic inhibitors:** a) Nevirapine (NVP); b) Efavirenz (EFV).

**Protease inhibitors:** a) Saquinavir (SQV); b) Indinavir (IDV); c) Nelfinavir (NFV).

Clinical isolated of resistant strains were obtained from patients characterised by a viro-immunological failure, observed using the standardised protocol of the AIDS Clinical Trials Group (ACTG).

All the patients showed the failure of the HAART therapy (two nucleosidic inhibitor *plus* a non-nucleosidic and/or a proteases inhibitor).

Virological failure was defined as either the increase of 0.5 logs copies/mL of the plasmatic level of HIV-RNA at the nadir or the increase of more than 10.000 copies/mL during the therapy for at least 12 weeks.

The patients received a HAART therapy for 24 month.

The cultures of PBMC obtained from healthy donors were co-cultivated with the same number of PBMC obtained from patient with HIV infection-disease at different level of viro-immunological failure.

The supernatant of the co-cultivated cultures was tested every four days to verify the production of p24 and to get the contextual isolation.

For the titration of every isolated viral strain, the dose infecting the 50% of the cultures (TCDI 50) was calculated using the method of Reed and Muench by the protocol said above.

The genotype of both the reverse transcriptase gene and of the protease gene was determined by direct sequencing and the method of the dideoxinucleotides chain termination, using a kit, which is commercially available (HIV genotyping System - Applied Byosystem) and an automatic sequencer ABI-PRISM 310 genetic Analyzer (Applied Byosystem).

For the phenotype of the nucleosidic, non-nucleosidic and proteases inhibitors was utilised a method which consider the use of human PBMC and the protocol standardised by the AIDS Clinical Trials Group, after verifying the genotype by sequence analysis.

In order to assay *in vitro* the susceptibility to chloroquine and hydroxychloroquine of the viral strains, it was used the protocol of the ACTG which is characterised by the measure of the production of antigen p24 in strongly infected cells.

The susceptibility of the viral strains to chemotherapics was determined in cell cultures, prepared in 96 wells microplates, using, for the infection, 0.1 MOI.

The supernatant of the infected cultures (either in absence or in presence of different concentrations of chemotherapic) were tested for the production of p24 after 7 days, using on ELISA test which is commercially available.

The concentrations of chloroquine and hydroxychloroquine tested ranged from 0.5 to 15 uM. At the same time non-infected cells were incubated with concentrations of chloroquine and hydroxychloroquine ranging from 0 to 50 uM to evaluate the toxicity of the compound.

After 4 day from the infection, half the cellular medium was replaced with a medium containing the corresponding dilution of chloroquine and hydroxychloroquine. The percentage of inhibition of the HIV replication, induced by chloroquine and hydroxychloroquine, was determined, after 7 day from the infection, comparing the levels of p24 of this cultures and that of the control, infected and not treated with chloroquine and hydroxychloroquine.

Therefore, the E.C.50 of chloroquine and hydroxychloroquine expresses the concentration of chemotherapic that inhibits the 50% of production of p24 and the values were calculated using linear and non linear regression analysis (the significativity for the non linear regression was R² > 0.95).

In the same experimental conditions and in the same day, but with similar concentrations of chloroquine and hydroxychloroquine, it was evaluated the vitality of the infect cell cultures, and this was expressed as I.C.50.

I.C.50 (Inhibitory Concentration) indicates the concentration of the chemotherapic agent inhibiting the vitality of the cell cultures at a rate of 50%, when there is not infection.

The phenotype indicates the values of the relationship: E.C.50 isolated virus
E.C.50 HIV-IIIB virus

In the present invention, the resistance to the chemotherapic is defined, in the phenotypic test, by the values of the relationship E.C.50 isolated virus
E.C.50 HIV-IIIB > 6

SI (Selectivity Index) Indicates the values of the relationship: I.C.50 cell cultures
E.C.50 isolated virus

S.I. indicates the number of times I.C.50 (dose inhibiting cellular vitality at a rate of 50%) is higher than the E.C.50 of the chemotherapic.

**TABLE 1**

| Effect of chloroquine and hydroxychloroquine on HIV clinical isolated, characterised by mutations in the RT gene inducing geno-phenotypic resistance to AZT. | | | | | | | | |
|---|---|---|---|---|---|---|---|---|
| Gen. | Cells | Fen AZT (fold)* | E.C.50 CQ (uM) | E:C.50 HCQ (uM) | S.I. CQ | S.I. HCQ | CQ (fold)* | Fen. HCQ (fold)* |
| D 67 Ns | PBMC | >80X | 3 | 2.5 | 3 | 3.6 | 2X | 1.6X |
| K 70N | | | | | | | | |
| T215F | | | | | | | | |
| K219Q | | | | | | | | |
| M41LD | PBMC | >80X | 3 | 2.8 | 3.2 | 3.4 | 1.6X | 1.5X |
| 76N K70R | | | | | | | | |
| T215Y | | | | | | | | |
| M41L | PBMC | >80X | 2.5 | 2.5 | 3.6 | 3.6 | 1.3X | 1.3X |
| D67N | | | | | | | | |
| K70RM | | | | | | | | |
| 184V | | | | | | | | |
| T215F | | | | | | | | |
| M41I | PBMC | 40X | 2 | 2.5 | 4 | 4.5 | 1.1X | 1.3X |
| T215Y | | | | | | | | |
| D67N | PBMC | 20X | 2 | 2 | 4 | 4 | 1.1X | 1.1X |
| K70R | | | | | | | | |
| T215F | | | | | | | | |
| K219Q | | | | | | | | |
| K70R | PBMC | 20X | 1.8 | 2 | 5.5 | 5 | 1.1X | 1.1X |
| T215F | | | | | | | | |
| K219Q | | | | | | | | |
| M41I | PBMC | 20X | 1.5 | 1 | 6 | 7 | 0.8X | 0.5X |
| M184V | | | | | | | | |
| L210W | | | | | | | | |
| R221K | | | | | | | | |
| T215Y | | | | | | | | |

| | | | | | | | | |
|---|---|---|---|---|---|---|---|---|
| * Number of times the drug concentration is higher than virus EC.50 considering the virus wild type. | | | | | | | | |

**TABLE 2**

| Effect of chloroquine and hydroxychloroquine on HIV clinical isolated,, characterised by mutations in the RT gene inducing geno-phenotypic resistance to ddI. | | | | | | | | |
|---|---|---|---|---|---|---|---|---|
| Gen | Cells | Fen. AZT (fold)* | E.C.50 CQ (uM) | E.C.50 HCQ (uM) | S.I. CQ | S.I. HCQ | Fen. CQ (fold) | Fen HCQ (fold)* |
| D67N | PBMC | >80X | 3 | 2.5 | 3 | 3.4 | 1.3X | 1.1X |
| K70R | | | | | | | | |
| T215F | | | | | | | | |
| K70R | | | | | | | | |
| M41L | PBMC | .>80X | 3 | 2.8 | 3.2 | 3.4 | 1.2X | 1.1X |
| D67N | | | | | | | | |
| K70R | | | | | | | | |
| T215Y | | | | | | | | |
| M41L | PBMC | >80X | 2.5 | 2.5 | 3.6 | 3.6 | 1.3X | 1.3X |
| D67N | | | | | | | | |
| K70R | | | | | | | | |
| M184V | | | | | | | | |
| T215Y | | | | | | | | |
| M41I | PBMC | 40X | 2 | 2.5 | 4 | 4.5 | 1.1X | 1.3X |
| T215F | | | | | | | | |
| D67N | PBMC | 40X | 2.5 | 2.5 | 4.5 | 4.5 | 1.5X | 1.3X |
| K70R | | | | | | | | |
| M184V | | | | | | | | |
| T215Y | | | | | | | | |
| K70R | PBMC | 20X | 1.8 | 2 | 5.5 | 5 | 0.7X | 0.8X |
| T215F | | | | | | | | |
| K219Q | | | | | | | | |
| M41L | PBMC | 20X | 2 | 2.5 | 4.5 | 4 | 1.1X | 1.3X |
| M184V | | | | | | | | |
| L210W | | | | | | | | |
| R211KL | | | | | | | | |
| 214F | | | | | | | | |
| T215Y | | | | | | | | |

| | | | | | | | | |
|---|---|---|---|---|---|---|---|---|
| * Number of times the drug concentration is higher than EC.50 considering the virus wild type. | | | | | | | | |

**TABLE 3**

| Effect of chloroquine and hydroxychloroquine on HIV-1 clinical isolated, characterised by mutations in the RT gene inducing geno-phenotypic resistance to 3TC. | | | | | | | | |
|---|---|---|---|---|---|---|---|---|
| Gen. | Cells | Fen. 3TC (fold) * | E.C.50 CQ (uM) | E.C.50 HCQ (uM) | S.I. CQ | S.I. HCQ | Fen. CQ (fold)* | Fen. HCQ (fold)* |
| M184V | PBMC | >80X | 3.2 | 3 | 2.5 | 3.2 | 1.4X | 1.3X |
| M184I | PBMC | >80X | 2.5 | 2.8 | 3.6 | 3.4 | 1.1X | 1.2X |
| M41L | PBMC | >80X | 3.5 | 3.2 | 3.4 | 3.3 | 1.9X | 1.7X |
| D67N | | | | | | | | |
| V118I | | | | | | | | |
| M184V | | | | | | | | |
| T215Y | | | | | | | | |
| M41L | PBMC | 40X | 3 | 2.5 | 3 | 3.5 | 2.1X | 1.5X |
| E44D | | | | | | | | |
| D67N | | | | | | | | |
| M184V | | | | | | | | |
| R211K | | | | | | | | |
| T215Y | | | | | | | | |

| | | | | | | | | |
|---|---|---|---|---|---|---|---|---|
| * Number of times the drug concentration is higher than E.C.50 considering the virus wild type. | | | | | | | | |

**TABLE 4**

| Effect of chloroquine and hydroxychloroquine on HIV-1 clinical isolated, characterised by mutations in the RT gene inducing geno-phenotypic resistance to d4T. | | | | | | | | |
|---|---|---|---|---|---|---|---|---|
| Gen. | Cells | Fen. D4T (fold)* | E.C.50 CQ (uM) | E.C.50 HCQ (uM) | S.I. CQ | S.I. HCQ | Fen. CQ (fold)* | Fen. HCQ (fold)* |
| K65R | PBMC | 40X | 3.5 | 3 | 3.4 | 3.3 | 2.3X | 2.2X |
| F116Y | | | | | | | | |
| Q151M | | | | | | | | |
| K65R | PBMC | 40X | 3.5 | 3.5 | 3.4 | 3.3 | 2.3X | 2.3X |
| F77L | | | | | | | | |
| Y115F | | | | | | | | |
| Q151M | | | | | | | | |
| M184V | | | | | | | | |
| V75I | PBMC | 20X | 2.2 | 3 | 3 | 4 | 1.5X | 2X |
| F77L | | | | | | | | |
| F116Y | | | | | | | | |
| Q151M | | | | | | | | |
| T69SSS | PBMC | 10X | 2.8 | 3 | 3.4 | 3.3 | 1.8X | 2X |
| T215Y | | | | | | | | |
| M41I | PBMC | 10X | 2.8 | 2.5 | 3.4 | 3.6 | 1.8X | 1.6X |
| T69SSS | | | | | | | | |
| T215Y | | | | | | | | |

| | | | | | | | | |
|---|---|---|---|---|---|---|---|---|
| * Number of times the drug concentration is higher than E.C.50 considering the virus wild type. | | | | | | | | |

**TABLE 5**

| Effect of chloroquine and hydroxychloroquine on HIV-1 clinical isolated, characterised by mutations in the RT gene inducing geno-phenotypic resistance to ABC. | | | | | | | | |
|---|---|---|---|---|---|---|---|---|
| Gen. | Cells | Fen. ABC (fold)* | E.C.50 CQ (uM) | E.C.50 HCQ (uM) | S.I. CQ | S.I. HCQ | Fen. CQ (fold)* | Fen. HCQ (fold)* |
| K65R | PBMC | 40X | 2.5 | 2.2 | 3.6 | 4 | 1.6X | 1.4X |
| S68G | | | | | | | | |
| F77L | | | | | | | | |
| Y115F | | | | | | | | |
| Q151M | | | | | | | | |
| M184V | | | | | | | | |
| K65R | PBMC | 10X | 1.8 | 1.5 | 5.5 | 6 | 1.2X | 1X |
| L74V | | | | | | | | |
| M184V | | | | | | | | |
| L74V | PBMC | 10X | 2.2 | 2.5 | 4 | 3.6 | 1.2X | 1.3X |
| Y115F | | | | | | | | |
| M184V | | | | | | | | |
| L74V | PBMC | 10X | 1.8 | 2.2 | 5.5 | 4 | 1.2X | 1.4X |
| M184F | | | | | | | | |
| M41L D67N | PBMC | 10X | 2.2 | 1.8 | 4 | 5.5 | 1.4X | 1.2X |
| M184V | | | | | | | | |
| L210W | | | | | | | | |
| T215Y | | | | | | | | |

| | | | | | | | | |
|---|---|---|---|---|---|---|---|---|
| * Number of times the drug concentration is higher than E.C.50 considering the virus wild type. | | | | | | | | |

**TABLE 6**

| Chloroquine and hydroxychloroquine activity towards viral replication of HIV-1 strains geno-phenotypically resistant to NVP. | | | | | | | | |
|---|---|---|---|---|---|---|---|---|
| Gen. | Cells | Fen. NVP (fold)* | E.C.50 CQ | E.C.50 HCQ | S.I. CQ | S.I. HCQ | Fen. CQ (fold)* | Fen. HCQ (fold)* |
| K103N | MT-2 | >80X | 3.2 | 2.8 | 6.2 | 7.8 | 1.7X | 1.5X |
| V106A | MT-2 | >80X | 2.8 | 3.2 | 7.8 | 6.2 | 1.5X | 1.7X |
| Y181C | MT-2 | >80X | 3.8 | 3.2 | 6 | 6.1 | 2.1X | 1.7X |
| G190E | MT-2 | >80X | 3.5 | 3.5 | 6 | 6 | 1.9X | 1.9X |

| | | | | | | | | |
|---|---|---|---|---|---|---|---|---|
| * Number of times the drug concentration is higher than E.C.50 considering the virus wild type. | | | | | | | | |

**TABLE 7**

| Effect of chloroquine and hydroxychloroquine on viral replication of HIV-1 strains geno-phenotypically resistant to EFV. | | | | | | | | |
|---|---|---|---|---|---|---|---|---|
| Gen. | Cells | Fen. EFV (fold)* | E.C.50 CQ (uM) | E.C.50 HCQ (uM) | S.I. CQ | S.I. HCQ | Fen. CQ (fold)* | Fen. HCQ (fold)* |
| L100I | MT-2 | >80X | 3.2 | 3.2 | 6.2 | 6 | 2.1X | 2.1X |
| K103N | | | | | | | | |
| K101D | MT-2 | >80X | 2.8 | 2.5 | 7.8 | 8.8 | 1.8X | 1.6X |
| K103N | | | | | | | | |
| K103N | MT-2 | >80X | 2.2 | 2.8 | 6.8 | 7.8 | 1.4X | 1.8X |
| V108D | | | | | | | | |
| K103N | MT-2 | >80X | 2.5 | 2.5 | 8 | 8.5 | 1.6X | 1.6X |
| Y181C | | | | | | | | |
| K103N | MT-2 | >80X | 2.2 | 2.8 | 8.5 | 8.7 | 1.4X | 1.8X |
| Y181C | MT-2 | 20X | 1.8 | 1.5 | 8.5 | 8.7 | 1.2X | 1X |

| | | | | | | | | |
|---|---|---|---|---|---|---|---|---|
| * Number of times the drug concentration is higher than E.C.50 considering the virus wild type. | | | | | | | | |

**TABLE 8**

| Effect of chloroquine and hydroxychloroquine on viral replication of HIV-1 clinical isolated, characterised by mutations in the protease gene inducing geno-phenotypic resistance to SQV | | | | | | | | |
|---|---|---|---|---|---|---|---|---|
| Gen. | Cells | Fen. SQV (fold)* | E.C.50 CQ (uM) | E.C.50 HCQ (uM) | S.I. CQ | S.I. HCQ | Fen. CQ (fold)* | Fen. HCQ (fold)* |
| G48V | PBMC | >80X | 3.5 | 3 | 3.4 | 3 | 1.9X | 1.6X |
| V82A | | | | | | | | |
| I84V | PBMC | >80X | 2.5 | 2.8 | 3.6 | 3 | 1.3X | 1.5X |
| L90M | | | | | | | | |
| G48V | PBMC | 40X | 1.8 | 1.5 | 5.5 | 4 | 1X | 0.8X |
| I84V | | | | | | | | |
| A74V | | | | | | | | |
| L90M | | | | | | | | |
| V82A | PBMC | 20X | 1.5 | 1.8 | 6 | 5.5 | 0.8X | 1X |
| L90M | | | | | | | | |
| M46I | PBMC | 20X | 2 | 1.8 | 6.7 | 6 | 1.1X | 1X |
| G48V | | | | | | | | |
| L63P | | | | | | | | |
| L90M | | | | | | | | |

| | | | | | | | | |
|---|---|---|---|---|---|---|---|---|
| . * Number of times the drug concentration is higher than E.C.50 considering the virus wild type. | | | | | | | | |

**TABLE 9**

| Effect of chloroquine and hydroxychloroquine on viral replication of HTV-1 clinical isolated, characterised by mutations in the protease gene inducing geno-phenotypic resistance to IDV. | | | | | | | | |
|---|---|---|---|---|---|---|---|---|
| Gen. | Cells | Fen. IDV (fold)* | E.C.50 CQ (uM) | E.C.50 HCQ (uM) | S.I. CQ | S.I. HCQ | Fen. CQ (fold)* | Fen. HCQ (fold)* |
| V82F | PBMC | >80X | 2.8 | 3.3 | 3.4 | 2.5 | 1.5X | 1.7X |
| L90M | | | | | | | | |
| G48V | PBMC | >80X | 2.5 | 2.8 | 3.6 | 3 | 1.3X | 1.5X |
| V82A | | | | | | | | |
| I84V | PBMC | 20X | 2.2 | 1.8 | 4 | 5.5 | 1.2X | 1X |
| L90M | | | | | | | | |
| L10R | PBMC | 10X | 1.5 | 1.8 | 5.5 | 5 | 1X | 0.8X |
| M46I | | | | | | | | |
| L63P | | | | | | | | |
| V82T | | | | | | | | |
| K20R | PBMC | 10X | 1.8 | 2.2 | 5.5 | 4.5 | 1X | 1.2X |
| E35D | | | | | | | | |
| M36I | | | | | | | | |
| I54V | | | | | | | | |
| V82T | | | | | | | | |

| | | | | | | | | |
|---|---|---|---|---|---|---|---|---|
| * Number of times the drug concentration is higher than E.C.50 considering the virus wild type. | | | | | | | | |

**TABLE 10**

| Effect of chloroquine and hydroxychloroquine on viral replication of HIV-1 clinical isolated, characterised by mutations on the protease gene inducing geno-phenotypic resistance to NFV. | | | | | | | | |
|---|---|---|---|---|---|---|---|---|
| Gen. | Cells | Fen. NFV (fold)* | E.C.50 CQ (uM) | E.C.50 HCQ (uM) | S.I. CQ | S.I. HCQ | Fen. CQ (fold)* | Fen. HCQ (fold)* |
| V82F | PBMC | >80X | 2.8 | 2.5 | 3.4 | 3.6 | 1.5X | 1.3X |
| L90M | | | | | | | | |
| G48V | PBMC | >80X | 3 | 3 | 3.2 | 3.3 | 1.6X | 1.6X |
| V92A | | | | | | | | |
| D30N | PBMC | 30X | 1.8 | 2.2 | 5.5 | 4 | 1X | 1.2X |
| A71V | | | | | | | | |
| D30N | PBMC | 30X | 2.2 | 1.8 | 4 | 5.5 | 1.2X | 1X |
| M46V | | | | | | | | |
| A71V | | | | | | | | |
| V82A | H-9 | 20X | 2.7 | 2.5 | 5 | 5.5 | 1.5X | 1.3X |
| D30N | H-9 | 30X | 2.5 | 2.2 | 4.2 | 4.4 | 1.3X | 1.2X |

| | | | | | | | | |
|---|---|---|---|---|---|---|---|---|
| * Number of times the drug concentration is higher than E.C.50 considering the virus wild type. | | | | | | | | |

**TABLE 11**

| Effect of chloroquine and hydroxychloroquine on the replication of HTV-1 strains cultivated in PBMC and isolated from patient naive to the treatment. | | | | | |
|---|---|---|---|---|---|
| Virus | Cells | E.C.50 CQ (uM) | E.C.50 HCQ (uM) | S.I. CQ | S.I. HCQ |
| N°1 | PBMC | 1.5 | 2.2 | 5 | 4.5 |
| N°2 | PBMC | 2.2 | 1.8 | 4 | 5 |
| N°3 | PBMC | 1.8 | 1.5 | 5 | 6 |
| N°4 | PBMC | 2.5 | 2.2 | 3.5 | 4 |
| N°5 | PBMC | 2 | 1.8 | 4 | 4.5 |

In each experiment which was carried out, the E.C.50 of chloroquine and hydroxychloroquine *versus* the HTV-1 virus sensitive to the anti-retroviral treatment ranged from 1.5 to 3.5 uM.

The E.C.50 of chloroquine and hydroxychloroquine *versus* the viruses resistant to both reverse transcriptase and protease inhibitors ranged from 1.5 to 3.5 uM.

The present invention provide for the use of at least one 4 amino-quinolinic derivative to obtain a drug for the anti-retroviral therapy.

In particular, said drug is carried out using either chloroquine or hydroxychloroquine or combinations of these two.

The therapy considers one or more oral administrations a day of said drug, which can be also administered in combination with other drugs showing anti-retroviral action.

The drug allows an administration of chloroquine ranging, in the average adult man, from about 200 to about 400 mg/day *per os* or an administration of hydroxychloroquine ranging, in the average adult man, from about 200 to about 600 mg/day *per os.*

Considering an anti-retroviral therapy based only on chloroquine, its administration ranges, in the average adult man, from about 300 to 400 mg/day *per os,* whereas considering an anti-retroviral therapy based only on hydroxychloroquine, the administration of this last ranges, in the average adult man, from about 400 to 600 mg/day *per os.* The drug allows reaching, in the average adult man, average plasmatic concentrations of said 4 amino-quinolinic derivatives equal to about 7-15 uM/mL. In plasma of said subjects there are concentrations of chloroquine and hydroxychloroquine 2-10 times higher than that required in order to inhibit the 50% of the viral replication in every studied resistant strain, which represent 90% of strains clinically observable after the HAART therapy failure.

Said results can be interpreted considering that chloroquine and hydroxychloroquine have an anti-viral action completely different with respect to that of the other anti-retroviral drugs currently used in HAART therapy.

Considering a therapy based on the administration of drugs, known for the anti-retroviral therapy, in combination either with chloroquine or with hydroxychloroquine, the administration, in the average adult man, ranges from about 200 to 400 and from about 200 to 600 mg/day *per os,* respectively.

The invention provides that the therapy is for the treatment of the retrovirus infections, in particular for the HIV infection-disease produced by sensitive, partially sensitive and resistant to known therapies viral strains.

The alteration of the glycosilated portion of the gp120 induced by chloroquine, hydroxychloroquine and 4-amino-quinolinic derivatives, determines, as previously examined, both a reduction of the replication and a decrease of the HIV infectivity; this would explain the good anti-retroviral activity that chloroquine and hydroxychloroquine have towards viruses resistant to both nucleosidic and non-nucleosidic reverse transcriptase inhibitors and to proteases inhibitors, analysed in the research which is the source of the present invention.

The present invention introduces the therapeutic use of compounds derived from the chloroquine and from the hydroxychloroquine shown as example in following graphic tables 12 and 13.

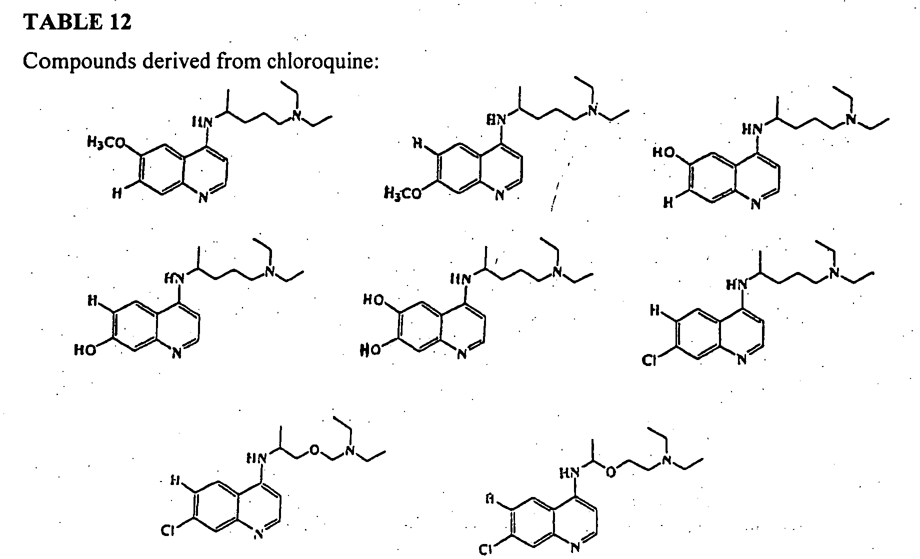

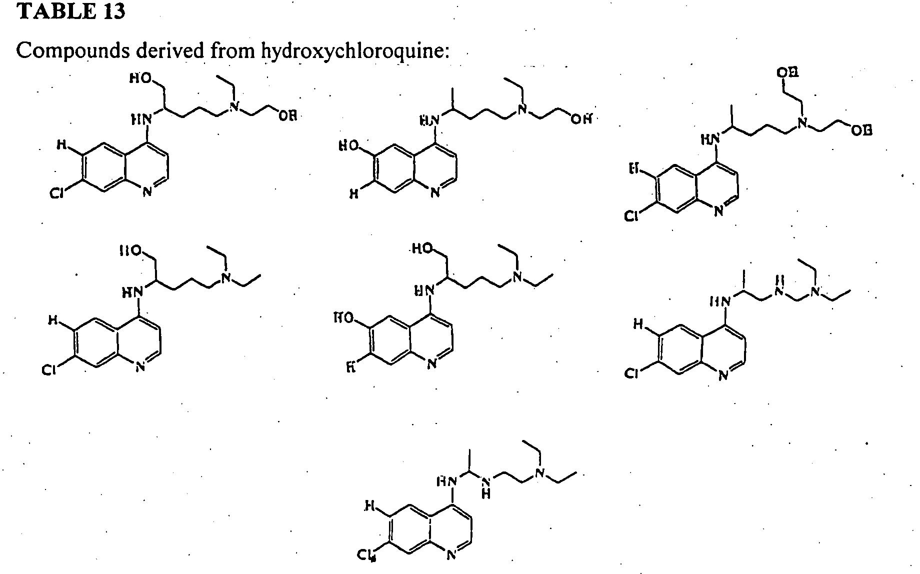

In particular, the invention provides the therapeutic use of chloroquine, hydroxychloroquine and of the derivatives of these two last, shown, as an example, in tables 12 and 13, as either right-handed or left-handed compounds or racemates, which possess specific and diversified anti-retroviral activities.

The invention provides the administration of chloroquine and hydroxychloroquine derived compounds, either pure or in any possible combination of different of them and with chloroquine and hydroxychloroquine, according to dosages similar to those above illustrated.

The main advantage of the present invention is to provide precise information about the use of chloroquine, hydroxychloroquine and 4 amino-quinolinic derivatives in the anti-retroviral therapy, through the preparation of a drug characterised by an optimal absorption when orally administrated, and by in *vivo* pharmacokinetic, pharmacodynamic and toxicity perfectly known.

The analysis of our data clearly shows that chloroquine and hydroxychloroquine and 4-amino-quinolinic derivatives are chemotherapics able to control contextually and with a good effectiveness the replication of HIV strains sensitive and resistant to both nucleosidic and non-nucleosidic reverse transcriptase inhibitors and to proteases inhibitors.

Chloroquine, hydroxychloroquine and 4-amino-quinolinic derivatives' inhibit a different phase during the HIV replication and, to be precise, the maturation of virions (structural alterations in the glycosilated portion of the gp 120 of the viral pericapsid).

The profile of acute and chronic toxicity is markedly better known in comparison to all the known anti-retroviral drugs, as chloroquine and hydroxychloroquine have been used in the therapy and the prophylaxis of malaria and against rheumatic diseases, in this case from more than 50 years and for long periods of time as, for example, for rheumatoid arthritis (16-18 months of daily therapy with 600 mg/day of hydroxychloroquine).

Further advantages are that chloroquine and hydroxychloroquine are widely available and the cheapest among chemotherapics that possess anti-HIV activity and that they have an inhibiting effect on the syntheses of the pro-inflammatory cytochines IL-1 and IL-6, which favour viral replication.

Moreover, chloroquine and hydroxychloroquine show *in vivo* and *in vitro* activities towards opportunist pathogenic agents correlated to the AIDS.

What has been above described is by way of example and it should not be limitative; for this, any possible variant in the formulation or dosage should be included in the present innovation, as above described and as claimed below.

## Claims

1. Use of at least one 4 amino-quinolinic derivative to obtain a drug **characterised in that** said drug is for the anti-retroviral therapy.

2. Use of at least one 4 amino-quinolinic derivative according to claim 1 **characterised in that** said derivative is chloroquine.

3. Use of at least one 4 amino-quinolinic derivative according to claim 1 **characterised in that** said derivative is hydroxychloroquine.

4. Use of at least one 4 amino-quinolinic derivative according to claim 1 **characterised in that** it includes at least one compound derived from chloroquine.

5. Use of at least one 4 amino-quinolinic derivative according to claim 1 **characterised in that** it includes at least one compound derived from hydroxychloroquine.

6. Use of at least one 4 amino-quinolinic derivative according to claim 1 **characterised in that** said derivative is a left-handed compound.

7. Use of at least one 4 amino-quinolinic derivative according to claim 1 **characterised in that** said derivative is a right-handed compound.

8. Use of at least one 4 amino-quinolinic derivative according to claim 1 **characterised in that** said derivative is a racemate.

9. Use of at least one 4 amino-quinolinic derivative according to one of the claims from 1 to 8 **characterised in that** said drug is administered once or several times a day.

10. Use of at least one 4 amino-quinolinic derivative according to claim 9 **characterised in that** said drug is included in a preparation for oral administration.

11. Use of at least one 4 amino-quinolinic derivative according to claim 1 **characterised in that** said drug can be administered in combination with other drugs showing an anti-retroviral action.

12. Use of at least one 4 amino-quinolinic derivative according to claim 2 **characterised in that** said drug allows, in the average adult man, an administration of chloroquine ranging from about 200 to about 400 mg/day *per os.*

13. Use of at least one 4 amino-quinolinic derivative according to claim 12 **characterised in that**, considering an anti-retroviral therapy based only on chloroquine, the administration ranges, in the average adult man, from about 300 to 400 *mg*/*day per os.*

14. Use of at least one 4 amino-quinolinic derivative according to claim 12 **characterised in that**, considering a therapy based on the administration of drugs known for anti-retroviral therapy in combination with chloroquine, the administration of the latter ranges, in the average adult man, from about 200 to 400 mg/day *per os.*

15. Use of at least one 4 amino-quinolinic derivative according to claim 3 **characterised in that** said drug allows an administration of hydroxychloroquine, in the average adult man, ranging from about 200 to about 600 mg/day *per os.*

16. Use of at least one 4 amino-quinolinic derivative according to the claim 15 **characterised in that**, considering an anti-retroviral therapy based only on hydroxychloroquine, the administration ranges, in the average adult man, from about 400 to 600 mg/day *per os.*

17. Use of at least one 4 amino-quinolinic derivative according to claim 15 **characterised in that**, considering a therapy based on the administration of drugs known for anti-retroviral therapy in combination with hydroxychloroquine, the administration of the latter ranges, in the average adult man, from about 200 to 600 mg/day *per os*.

18. Use of at least one 4 amino-quinolinic derivative according to claims 4 and 5 **characterised in that** said drug allows the administration of one or more compounds derived from chloroquine and/or from hydroxychloroquine ranging, in the average adult man, from about 100 to about 700 mg/day *per os*.

19. Use of at least one 4 amino-quinolinic derivative according to claim 1 **characterised in that** said drug allows to reach average plasmatic concentrations of said derivative equal to about 7-15 uM/mL.

20. Use of at least one 4 amino-quinolinic derivative according to each of preceding claims **characterised in that** the therapy is for treating HIV infection-disease.

21. Use of at least one 4 amino-quinolinic derivative according to each of preceding claims **characterised in that** the therapy is for treating HIV infection-disease partially or totally resistant to known therapies.
